# EUROPEAN PATENT APPLICATION

(11) **EP 2 774 588 A1**
(43) Date of publication of application: **10.09.2014**
(21) Application number: 14158422.7
(22) Date of filing: 07.03.2014
(51) Int. Cl.: A61F 13/08, A41D 27/28, A41B 11/00

(54) **Cut Resistant Compression Garment with Moisture Channelling**

(30) Priority: 08.03.2013 US 201361774765 P
(71) Applicant: Elicit Brands, LLC, Brentwood, TN 37027 (US)
(72) Inventor: Cleveland, Mark, Brentwood, TN Tennessee 37027 (US)
(74) Representative: Baldwin, Mark

(57) **Abstract**

A compression sock has a sock body including a top band, a sock front region, a sock rear region, and a sock bottom. A sweat ejection port is defined proximate the sock bottom. A sweat channel extends continuously from the top band to the ejection port. The sweat channel is made of a hydrophilic material embedded with ions effective to attract body sweat into the sweat channel. Separate cut-resistant front and rear shields may be integrated into front and rear regions of the sock.

## Description

A portion of the disclosure of this patent document contains material that is subject to copyright protection. The copyright owner has no objection to the reproduction of the patent document or the patent disclosure, as it appears in the U.S. Patent and Trademark Office patent file or records, but otherwise reserves all copyright rights whatsoever.

### CROSS-REFERENCES TO RELATED APPLICATIONS

This application claims benefit of U.S. Provisional Patent App. No. 61/774,765 filed March 8, 2013, entitled "Cut Resistant, Moisture Channelling Compression Sock" which is incorporated herein in its entirety.

### BACKGROUND OF THE INVENTION

The present invention relates generally to socks, sleeves and liners and other compression garments that are worn to cover parts of the extremities, such as the foot and ankle. More particularly, this invention pertains to compression socks, sleeves and liners that are worn by athletes to protect and support their feet and lower legs during training and athletic competitions. The present invention also pertains to liner socks worn by amputees who use prosthetics on their extremities.

Many sporting, outdoor and athletic activities involve risk of injury to the feet and lower legs of the participants. For example, participants in ice sports are routinely exposed to slashing wounds from an open skate blade. This makes the sport more dangerous. Therefore, protective gear has been developed for the safety of players. However, an area that is typically exposed but not sufficiently protected is the rear, lower and upper calf area commonly referenced as the Achilles tendon area and those tendons in the front of the calf between the skate boot tongue and shin guard.

The tradition of hockey includes skating without socks, or wearing skates that can be one or more full sizes smaller than the equivalent shoe size. Many conventional socks are not sufficiently thin as preferred by players who want to "feel the ice". A thin sock can be a difficult platform when managing moisture as an objective which means that sock odour is often an immediate problem. Also, socks worn by many athletes must be a compression sock to reduce swelling and improve both muscular support and venous return.

Socks currently available which promise to protect the lower leg from slashing wounds use aramid and other cut resistant materials. These conventional designs locate the cut resistant material in the sock in places where the benefit of protection is not obvious and the risk of sustaining a wound is minimal. Also, these cut resistant materials do not offer efficient moisture wicking properties and they are not as flexible or soft as may be desirable. Therefore, these products are uncomfortable for players to wear. There is no compression sock on the market that is also cut resistant. Furthermore, there are no sophisticated moisture management techniques used in any conventional sock.

Compression liner socks are often worn by amputees underneath a prosthetic liner. Moisture management is also needed in prosthetic liner socks to reduce blistering, sweat build-up and bacterial growth.

What is needed, then, are moisture-wicking compression garments, such as socks, liners and sleeves that are comfortable to wear and, when worn by hockey players and other athletes, provide effective injury protection to the wearer at the points most highly at risk.

### BRIEF SUMMARY OF THE INVENTION

In an embodiment, the present invention is a compression garment having a garment body with an upper section, a lower section, a sweat ejection port defined in the lower section, and at least one sweat channel extending continuously from the upper section of the garment body to the ejection port, the sweat channel being formed of a hydrophilic material. The sweat channel may be defined by sweat channel bands adjacent to the sweat channel, the sweat channel bands formed from a semi-hydrophobic material. In some embodiments, at least part of the garment body outside of the sweat channel may include a hydrophobic material. In some embodiments, the sweat ejection port may be defined by alternating sections of hydrophobic and hydrophilic materials.

In some embodiments, the compression garment may be an athletic sock, such as a hockey sock. In other embodiments, the compression garment may be a prosthetic liner sock.

In another aspect, the compression garment may have positively charged ions embedded in the sweat channel and negatively charged ions embedded in at least part of the garment body outside of the sweat channel. Further, an anti-microbial material may be embedded in at least a portion of the sock body.

In some embodiments, the upper section of the garment body may have a top band configured to collect and direct sweat moving downward toward the compression garment into the sweat channel.

According to other athletic sock embodiments of the invention, a rear portion of the sock body may include a cut-resistant material defining a rear protective shield positioned to protect at least part of the back of the foot when the sock is worn. Similarly, a front section of the sock body may include cut-resistant material defining a front protective shield positioned to protect at least parts of the ankle and shin when the sock is worn. The front and rear protective shields may be configured in the sock body such that when the sock is worn, each of the front and rear protective shields are located only at areas of the foot and leg that are exposed to contact, and so that each of the front and rear protective shields are discontinuous around the foot and leg to enable continuous flow of moisture through the sweat channel.

### BRIEF DESCRIPTION OF THE SEVERAL VIEWS OF THE DRAWINGS

Fig. 1 is a plan view of one embodiment of a compression sock according to the present invention.
Fig. 2 is an enlarged view of a bottom portion of the sock of Fig. 1, showing a lower region of the sweat channel and the ejection port.
Fig. 3 an enlarged view of a side ankle portion of the sock of Fig. 1, showing an upper section of the sweat channel and the ultra-thin ankle bone area of the sock.
Fig. 4 is an enlarged view of a side foot portion of the sock of Fig. 1, showing a lower section of the sweat channel.
Fig. 5 is an enlarged view of a front portion of the sock of Fig. 1, showing an upper section of the sweat channel and a front region of the protective shield.
Fig. 6 is an enlarged view of a front portion of the sock of Fig. 1, showing a front region of the protective shield that is proximate the ankle and shin of the wearer.
Fig. 7 is a side view of a knee-high embodiment of the sock of the present invention, as worn.
Fig. 8 is a front view of a knee-high embodiment of the sock of the present invention, as worn.
Fig. 9 is a rear view of a knee-high embodiment of the sock of the present invention, as worn
Fig. 10 is a side view of an ankle-high embodiment of the sock of the present invention, as worn.
Fig. 11 is a perspective view of an embodiment of prosthetic liner sock according to the present invention.

### DETAILED DESCRIPTION OF THE INVENTION

Managing large volumes of sweat in the lower leg and the sources of sweat in the upper body is a significant challenge. In one aspect, the present invention implements multiple unique strategies for managing sweat generated in and around a compression garment during use. These strategies include a combination of structures in the sock that: (a) retard downward progress of upper body sweat; (b) provide for wicking of sweat using polyolefin and other materials to maximize dryness in specific areas; and (c) direct the sweat generated into a sweat channel defined in the garment which leads to a specific ejection port in the sock and shoe/boot where it can be ejected, by forces in the boot and by the function of the materials at that point of the sock

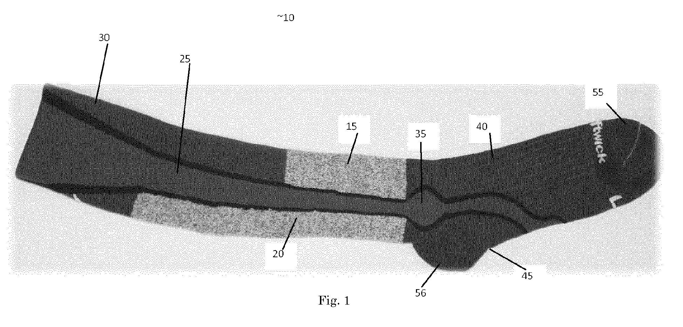

Referring to Figs. 1-8, a first embodiment of a knee-high compression sock 10 is shown. According to one aspect of the present invention, the sock 10 includes multiple functional structures integrated into a single sock. In the embodiment illustrated in Figs. 1-8, the compression sock 10 includes at least four novel structures integrated into the sock body: a front ankle/shin shield 15, a rear Achilles shield 20, a sweat channel 25, and a sweat ejection port 50.

In one embodiment of a hockey version of sock 10, a top band 30 is provided at the top of the sock 10. The top band 30 may be a four inch welt with a double layer construction to place an inner layer of comfortable olefin material against the skin surface. In some embodiments, the outer layer of the top band 30 can be made of a cut-resistant material. With this structure, the top band 30 provides both comfort and protection for the wearer. The top band 30 may also act as a "sweat dam" to help collect, manage and direct upper body sweat volume into the sweat channel 25.

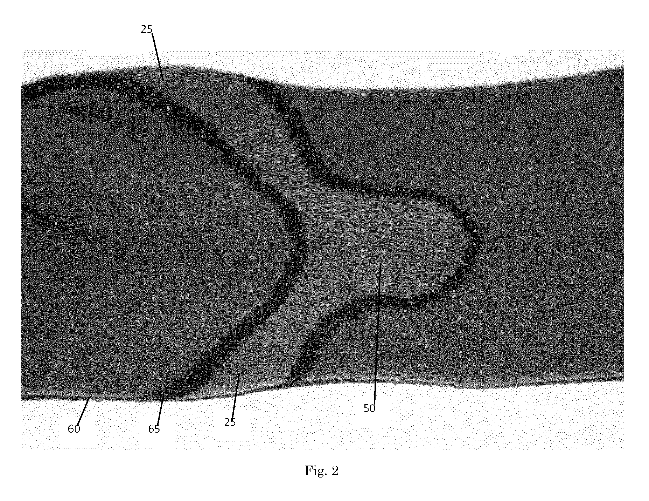

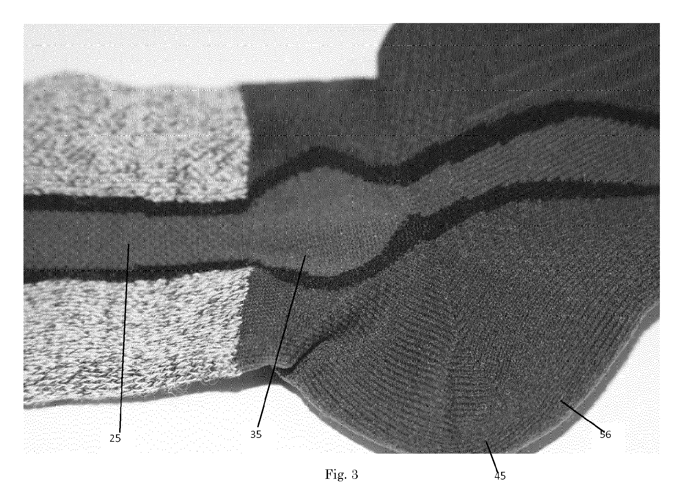

Looking at Fig. 2, most of the body of sock 10 is made of a hydrophobic material 60. The sweat channel 25 includes one or more continuous regions of hydrophilic material that collect and direct sweat downward along the sides of sock 10 until it reaches the ejection port 50 formed in the bottom of the sock 10. The sweat channel 25 may be defined by adjacent narrow regions 65 or sweat channel bands 65 made of a semi-hydrophobic material. The hydrophilic material in the sweat channel 25 can be formed as a 1x1 rib knit to facilitate both sweat flow and movement. Preferably, the sweat channel 25 extends continuously over the ankle bone region 35 of the sock 10 (Fig. 3) where the hydrophilic material may be thinned into a flat weave to provide greater comfort and to allow for continuous channelling of sweat.

The sweat channel 25 terminates at one or more ejection ports 50. In some hockey sock embodiments, the ejection port may be located in the bottom of the body of sock 10 so that when the sock is worn, the ejection port is positioned over or proximate to holes drilled in the skate body. This allows moisture to be evacuated from the tight confines of the skate boot. In some embodiments, the sweat ejection port 50 can be made of alternating squares or sections of hydrophobic and hydrophilic materials, such as in a 5x7 checkerboard pattern. Thus, the ejection port 50 functions to attract and hold moisture as long as the arch action in the boot does not press the moisture out. With this design, the foot anatomy acts as an engine to pump water out at a defined ejection port location rather than just spreading the moisture to the heel and toe region.

In some embodiments, the sock materials can include an embedded active anti-microbial to provide anti-odour and ant-fungal benefits. For example, silver ions can be bonded into the sock material. These ions may then be activated by contact with sweat. In some embodiments, both negatively and positively charged ions are used. The positively charged ions are positioned in the sock to attract sweat to the sweat channel 25. The negatively charged ions may be positioned in areas of the sock body adjacent to the sweat channel 25 to "repel' the sweat into the sweat channel 25.

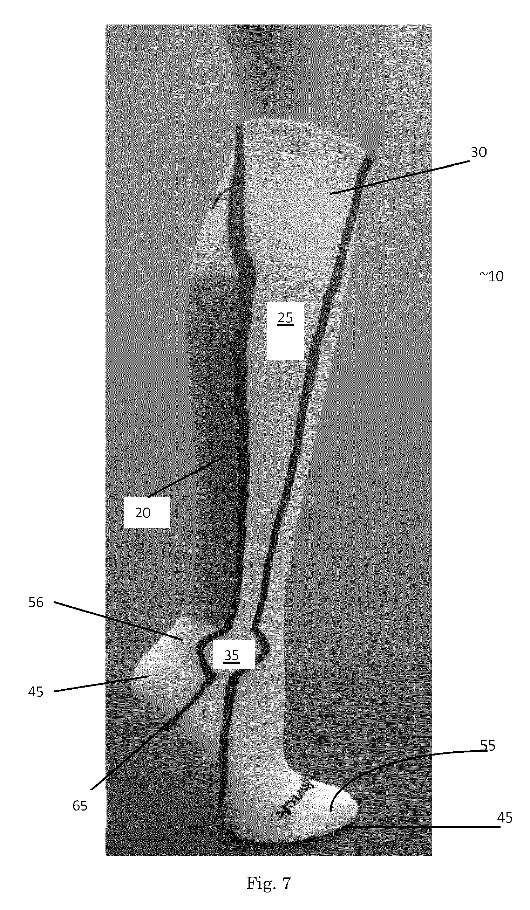

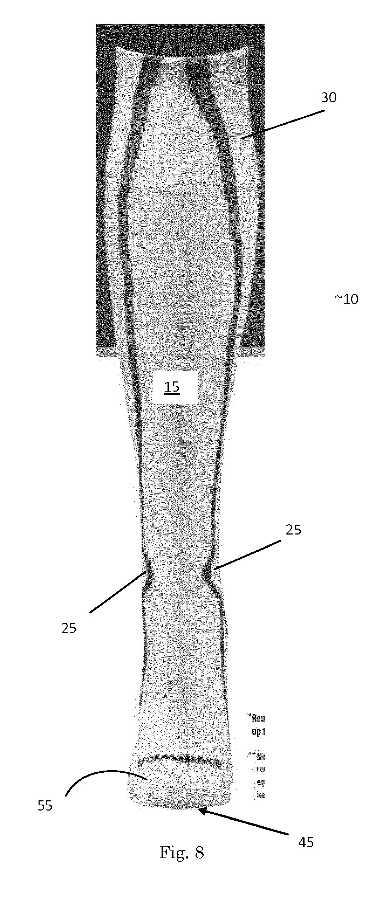

In some embodiments, a rear protective shield 20 (Fig. 7) may be integrated into the rear section of sock 10 to provide cut protection for the Achilles tendon and adjacent tissues of the foot, ankle and leg. A front protective shield 15 (Fig. 8) may be integrated into the front of sock 10 to provide cut protection for the shin and front of the ankle. In some embodiments, the front and rear protective shields 15 and 20 can be made of conventional cut-resistant fibres such as aramid. In other embodiments, the front and rear protective shields can be made of X-13 cut-resistant yarn supplied by Patrick Yarns of King Mountain, North Carolina.

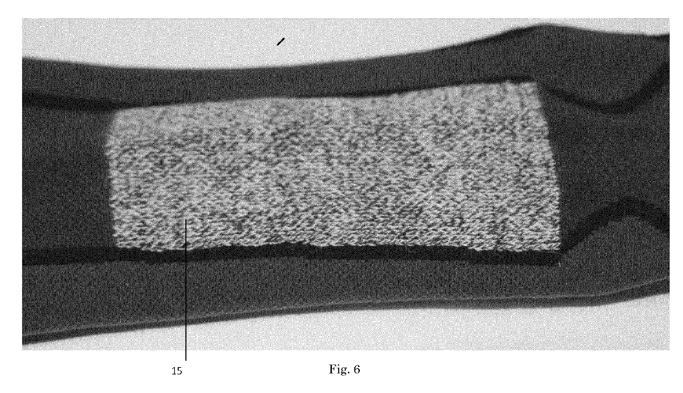

In some embodiments, the front and rear protective shields 15, 20 may be positioned in the sock 10 only at the exposed areas of the foot and leg and may not extend continuously around the foot and leg. For example, looking at the hockey sock embodiment of Fig. 6, the front protective shield 15 extends between an upper shin guard area to the skate boot area. This discontinuous configuration of the protective shields 15, 20 as shown allows the sweat channel 25 to extend continuously down either side of the foot and leg.

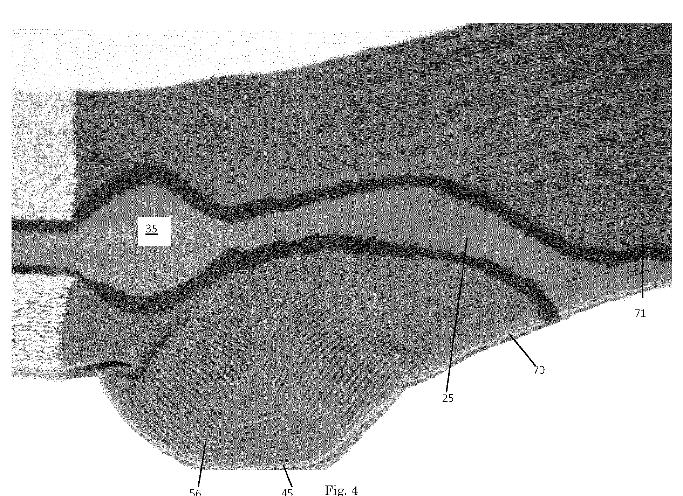

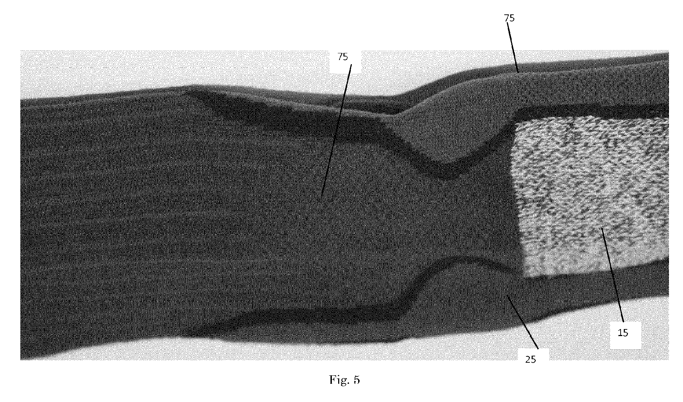

In some embodiments as shown in Fig. 1, the sock 10 may include padding 45 at the heel section 56 and toe section 55, and an arch support region 40. Also, to minimize bunching of the sock around the ankle, a half-density weave construction 75 may be used near the ankle, as shown in Fig. 5. Other areas of the sock may employ a full density olefin weave, as indicated at areas 70 and 71 on Fig. 4.

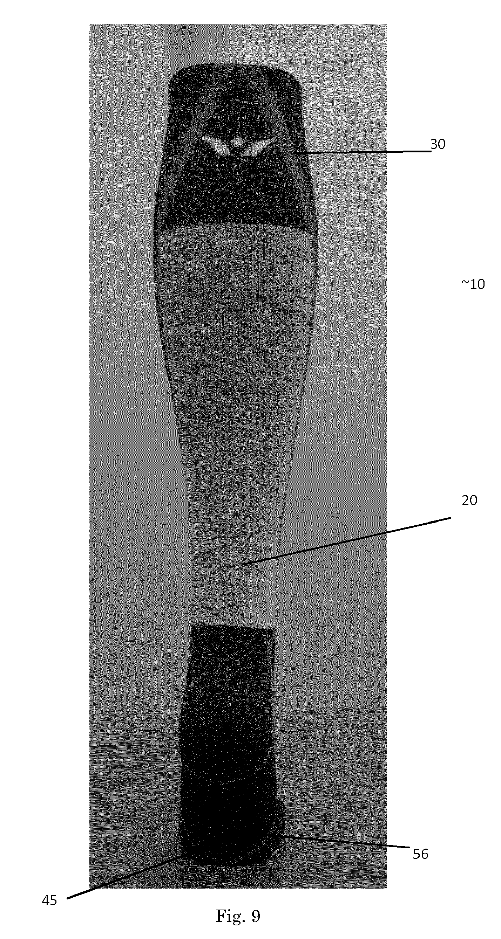

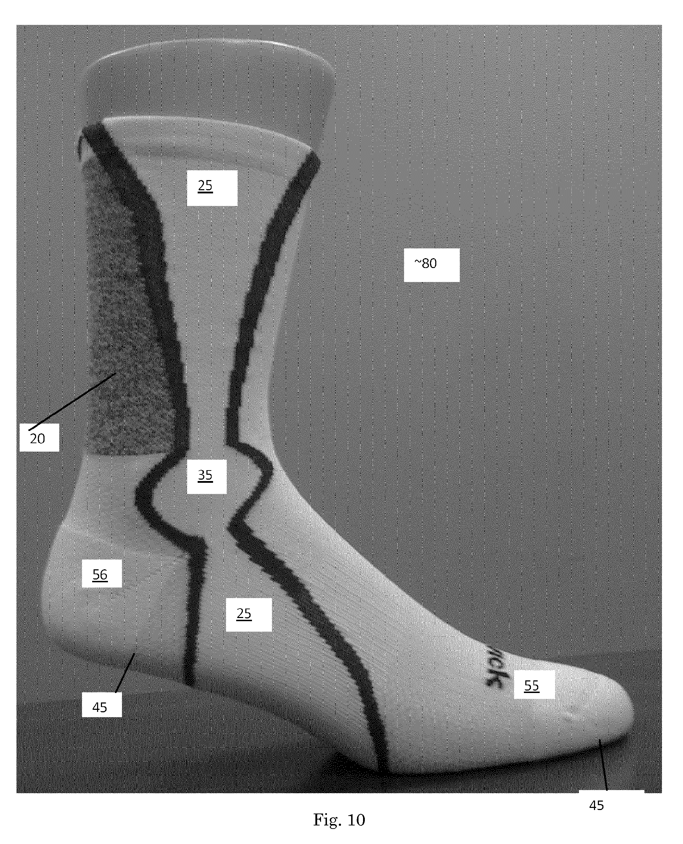

Fig. 9 illustrates a rear view of a knee-high embodiment of sock 10. Fig. 10 shows an ankle-length sock 80 according to another embodiment of the invention. In the embodiments shown in Figs. 9 and 10, the front protective shield 15 may be omitted.

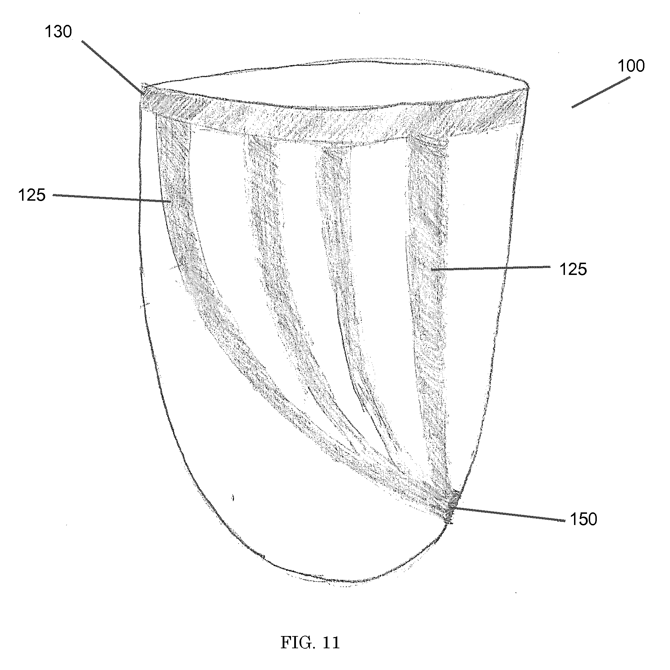

Fig. 11 shows another embodiment of a compression garment according to the present invention, configured as a prosthetic liner sock 100. The prosthetic liner sock 100 may be worn by an amputee at least partially underneath a prosthesis or under a liner for a prosthesis for comfort and moisture control. In this embodiment, multiple sweat channels 125 may be defined in the sock body. Each sweat channel 125 may be configured to extend continuously down the sock body and then terminate in a common ejection port 150. Alternatively, multiple ejection ports may be provided. A top band 130 may be provided around the top of the sock body to collect, manage, and direct moisture toward the sweat channels 125.

While embodiments of the compression garments are described herein as socks or prosthetic liner socks, other embodiments are also within the scope of the present invention, including compression sleeves worn on various parts of the upper and lower extremities.

The compression garment of the present invention as disclosed herein uniquely presents hydrophobic materials in a double layer, extended against the skin, purposed to absorb, repel and manage upper body sweat to prevent, as much as possible, collection of that source of sweat volume which otherwise descends into the boot. When upper body sweat volume overpowers the garment's first defence, it is then channelled to a specific point, using the sweat channel structures and methods as illustrated and described. The salty brine of sweat carries charge, and the garment materials may also carry a charge which repels sweat to a specific section (e.g., the sweat channel which is made from materials with a comparatively more attractive charge). These same sections of the sock are made with hydrophobic materials, and the hydrophobic differentials of different sock regions assist in channelling sweat to the ejection port.

The properties of these various materials also wick moisture, with the objective to maintain the most dry surface at the front and back of the sock where skates have binding surfaces on the tongue and heel section of the boot. Additionally, to present a dry, yet flexible surface at these points to improve the athlete's performance and comfort, the sock of the present invention does not have inflexible cut resistant materials descending into the boot. The channelling function of the sock should not be blocked by the cut resistant material.

Once sweat is in the boot, an objective of the present invention is to keep the heel and toe sections as dry as possible. The concept of ejecting excess sweat helps minimize moisture remaining in the sock and represents a novel strategy in moisture management. Holes drilled in the skate may be placed in any spot and the sock ejection system and port can be placed in a single or at multiple points.

The features of present invention may also affect the manufacturing process. The machines that make compression socks are constantly called upon to cut materials, change patterns and call new materials. Traditional cut resistant socks, by their nature, employ materials that are difficult to cut. A machine employed to construct a conventional cut-resistant sock will typically cut the material only once (or a highly limited number of times) within the program to build the sock. This is because it is hard on the machines and expensive to execute any design that requires frequent cuts. To enable and employ the sweat channel as described above, the present invention deploys a strategy where the cut resistant material is used only where needed and optimized against risk.

Thus, although there have been described particular embodiments of the present invention of a new and useful cut-resistant, moisture-channelling compression garment, it is not intended that such references be construed as limitations upon the scope of this invention except as set forth in the following claims.

## Claims

1. A compression garment (10; 80; 100) comprising:
a garment body having an upper section and a lower section;
a sweat ejection port (50; 150) defined in the lower section of the garment body;
a sweat channel (25; 125) defined in the garment body and extending continuously from the upper section of the garment body to the ejection port (50; 150), the sweat channel comprising a hydrophilic material.

2. The compression garment of claim 1, wherein:
the sweat channel (25; 125) is defined by sweat channel bands adjacent to the sweat channel, the sweat channel bands comprising a semi-hydrophobic material; and
at least part of the garment body outside of the sweat channel comprises a hydrophobic material.

3. The compression garment of claim 1 or 2, further comprising positively charged ions embedded in the sweat channel (25; 125).

4. The compression garment of claim 3, further comprising negatively charged ions embedded in at least part of the garment body outside of the sweat channel (25; 125).

5. The compression garment of any one of the preceding claims, further comprising an anti-microbial material embedded in at least a portion of the garment body.

6. The compression garment of claim 5, wherein the anti-microbial material comprises silver ions.

7. The compression garment of any one of the preceding claims, wherein the upper section of the garment body further comprises a top band (30; 130) configured to collect and direct sweat moving downward toward the compression garment into the sweat channel (25; 125).

8. The compression garment of claim 7, wherein the top band comprises a cut-resistant material.

9. The compression garment of any one of the preceding claims, wherein:
the garment body further comprises a rear section; and
the rear section of the garment body comprises a cut-resistant material defining a rear protective shield (20) positioned to protect at least part of the back of the foot when the garment is worn.

10. The compression garment of any one of claims 1 to 8, wherein:
the garment body further comprises a front section; and
the front section of the garment body comprises a cut-resistant material defining a front protective shield (15) positioned to protect at least parts of the ankle and shin when the garment is worn.

11. The compression garment of claim 10 wherein:
the garment body further comprises a rear section;
the rear section of the garment body comprises a cut-resistant material defining a rear protective shield (20) positioned to protect at least part of the back of the foot when the garment is worn; and
each of the front and rear protective shields (15, 20) are configured in the garment body such that
when the garment is worn, each of the front and rear protective shields are located only at areas of the foot and leg that are exposed to contact, and
each of the front and rear protective shields is discontinuous around the foot and leg to enable continuous flow of moisture through the sweat channel (25).

12. The compression garment of any one of the preceding claims, wherein the sweat ejection port (50; 150) comprises alternating sections of hydrophobic and hydrophilic materials.

13. The compression garment of any one of the preceding claims, wherein the garment body is configured as a compression athletic sock (10; 80).

14. The compression garment of any one of claims 1 to 8, wherein the garment body is configured as a prosthetic liner sock (100).

15. The compression garment of any one of the preceding claims, comprising a plurality of said sweat channels (25; 125) and/or a plurality of said sweat ejection ports (50; 150).
